(19) Europäisches Patentamt
European Patent Office
Office européen des brevets

(11) Veröffentlichungsnummer: **0 235 085**
**A1**

(12) # EUROPÄISCHE PATENTANMELDUNG

(21) Anmeldenummer: **87810088.2**

(22) Anmeldetag: **16.02.87**

(51) Int. Cl.[3]: **C 07 D 493/22**
A 01 N 43/90
//(C07D493/22, 313:00, 311:00, 311:00, 307:00)

(30) Priorität: **20.02.86 CH 674/86**

(43) Veröffentlichungstag der Anmeldung:
**02.09.87 Patentblatt 87/36**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel(CH)**

(72) Erfinder: **Frei, Bruno, Dr.**
**Bündtenstrasse 16**
**CH-4410 Liestal(CH)**

(72) Erfinder: **Mereyala, Hari Babu, Dr.**
**NCL-Colony, C-5**
**Pashan Pune- 411008(IN)**

(54) **13-Beta-Zuckerderivate von Milbemycinen, deren Herstellung und Verwendung gegen Ekto-und Endoparasiten am Nutztier oder an der Nutzpflanze.**

(57) Es werden parasitizid und insektizid hochaktive Wirkstoffe der Formel I

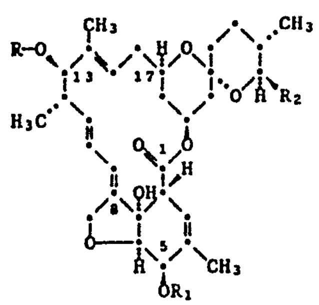

(I)

worin
$R_1$ Wasserstoff oder eine Schutzgruppe bedeutet;
$R_2$ für Methyl, Ethyl, Isopropyl oder sek.-Butyl steht; und
R für einen Zuckerrest steht, beschrieben, sowie ihre Herstellung ausgehend von den entsprechend substituierten 13β-HydroxiMilbemycinen.

**EP 0 235 085 A1**

0235085

CIBA-GEIGY AG
Basel (Schweiz)

5-15762/=

13β-Zuckerderivate von Milbemycinen, deren Herstellung und Verwendung gegen Ekto- und Endoparasiten am Nutztier oder an der Nutzpflanze

Die vorliegende Erfindung betrifft neue 13β-Zuckerderivate von Milbemycinen der nachstehenden Formel I, deren Herstellung sowie deren Verwendung zur Bekämpfung von Schädlingen wie Ekto- und Endoparasiten an Tieren oder Pflanzen.

Bei den erfindungsgemässen Verbindungen handelt es sich um 13β-Zuckerderivate von Milbemycinen mit der allgemeinen Formel I

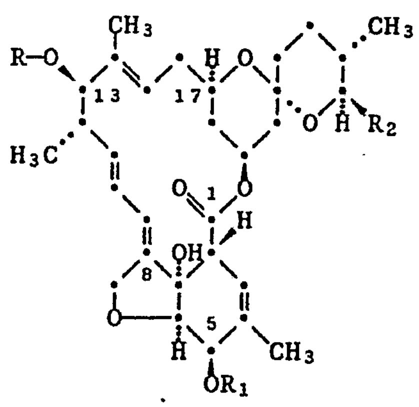

(I)

worin

$R_1$ Wasserstoff oder eine Schutzgruppe bedeutet;

$R_2$ für Methyl, Ethyl, Isopropyl oder sek.-Butyl steht; und

R für einen Zuckerrest steht.

Unter dem Begriff Alkyl selbst oder als Bestandteil eines anderen Substituenten sind je nach Zahl der angegebenen Kohlenstoffatome beispielsweise folgenden Gruppen zu verstehen: Methyl, Ethyl, Propyl, Butyl, Pentyl, Hexyl, Heptyl, Octyl, Nonyl, Decyl, usw. sowie die Isomeren, wie z.B. Isopropyl, Isobutyl, tert.-Butyl, Isopentyl usw.. Unter OH-Schutzgruppen für den Substituenten $R_1$ sollen hier und im folgenden die in der organischen Chemie üblichen Schutzfunktionen verstanden werden. Dabei handelt es sich insbesondere um Acyl- und Silylgruppen. Geeignete Acylgruppen sind beispielsweise die Reste $R_1-C(O)-$, worin $R_4$ die unter Formel I angegebenen Bedeutungen hat und vorzugsweise für $C_1-C_6$-Alkyl, $C_1-C_6$-Haloalkyl oder unsubstituiertes oder durch Halogen, $C_1-C_3$-Alkyl, $CF_3$ oder Nitro substituiertes Phenyl steht. Als geeignete Silylgruppen für $R_1$ kommt der Rest $-Si(R_5)(R_6)(R_7)$ in Frage, wobei $R_5$, $R_6$ und $R_7$ vorzugsweise unabhängig voneinander für $C_1-C_4$-Alkyl, Benzyl oder Phenyl stehen und beispielsweise eine der Gruppen Trimethylsilyl, tris(tert.Butyl)silyl, Diphenyl-tert.butylsilyl, bis(Isopropyl)methylsilyl, Dimethyl-(2,3-dimethyl-2-butyl)silyl, Triphenylsilyl usw. und insbesondere tert.Butyl-dimethylsilyl bildet. Die 5-OH-Gruppe kann auch als Benzylether oder Methoxiethoximethylether vorliegen.

Verbindungen, worin $R_2$ sek.Butyl darstellt, sollen hier und im folgenden gleichfalls zu den Milbemycin-Derivaten gerechnet werden, obwohl sie nach der üblichen Systematik nicht darunter fallen, sondern gemäss US-PS 4.173.571 von Avermectin-Derivaten abgeleitet sind.

Verbindungen der Formel I, worin $R_1$ eine Schutzgruppe darstellt, lassen sich durch einfache, z.B. hydrolytische Abspaltung der Schutzfunktion in die hochaktiven freien 5-Hydroxi-derivate ($R_1$=H) überführen und haben somit Zwischenprodukte-Charakter. Im übrigen wird der biologische Wert dieser Verbindungen durch die Schutzgruppe im Prinzip nicht gemindert.

Die Substituenten R' in 13-Position bedeuten in natürlich vorkommenden Milbemycinen ($R_1$ = H; $R_2$ = $CH_3$, $C_2H_5$ oder iso$C_3H_7$) stets Wasserstoff.

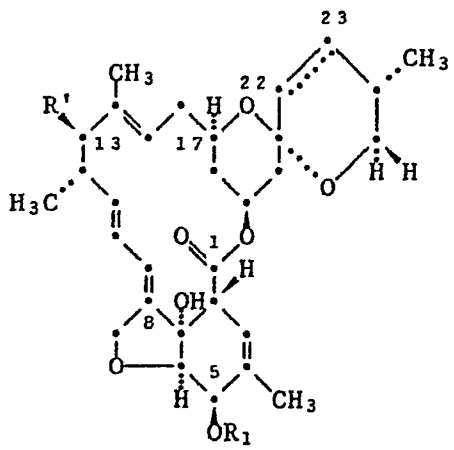

(Konstitution von natürlichen Milbemycinen)

Bei Avermectinen dagegen steht in der 13-Position als R' ein α-L-Oleandrosyl-α-L-oleandrose-Rest, der über Sauerstoff in α-Konfiguration mit dem Makrolid-Molekül verknüpft ist. Avermectine unterscheiden sich strukturell ausserdem durch eine 23-OH-Gruppe oder $\Delta^{22,23}$-Doppelbindung und in der Regel durch einen Substituenten $R_2$ = sek.$C_4H_9$ von den Milbemycinen. Durch Hydrolyse des Zucker-Restes der Avermectine gelangt man leicht zu den entsprechenden Avermectin-aglykonen, die eine allylische 13α-Hydroxy-Gruppe besitzen. Bei den Avermectin-derivaten der vorliegenden Anmeldung liegt die $\Delta^{22,23}$-Doppelbindung stets in hydrierter Form vor.

Formel I repräsentiert somit Milbemycin-Abkömmlinge, die in 5-Position entweder eine freie OH-Gruppe, eine Silyl- oder Acylgruppe aufweisen und in 13β-Position ein über ein Sauerstoff gebundenes Mono-, Di- oder Trisaccharid als Substituenten tragen.

Unter einem Zuckerrest soll im Rahmen der vorliegenden Erfindung vorzugsweise die Kohlenhydratgruppe $-A-(B)_m-(C)_n$ verstanden werden wobei A einen in 1'-Stellung verknüpften Kohlenhydratrest bedeutet, der glykosidisch an ein zweites oder drittes Kohlenhydratmolekül B und/oder C beliebiger Struktur geknüpft sein kann, wobei m und n unabhängig voneinander 0 oder 1 bedeuten.

Als Zuckerreste kommen somit in der Furanosyl- bzw. in der Pyranosylform vorliegend z.B. folgende Reste in Frage:

Monosaccharide: Glucose, Fructose, Altrose, Mannose, Sorbose, Gulose, Idose, Allose, Galactose, Ribose, Rhamnose, Arabinose, Xylose, Lyxose, Erythrose, Threose, Thamnose, Oleandrose, Altrose, Talose sowie ihre entsprechenden Derivate wie Methylglukose, Trimethylglukose und Tetraacetylglukose sowie ein- oder mehrfach O-acylierte bzw. O-alkylierte Zucker, ferner Deoxy-Zucker wie 2-Deoxy-glukose, 2-Deoxy-galactose, 2-Deoxy-Rhamnose und 2-Deoxyribose sowie ihre entsprechenden Derivate;

Disaccharide: Lactose, Maltose, Cellobiose, Melibiose, Gentiobiose, Oleandryl-oleandrose und beliebige Kombinationen von zwei der oben aufgeführten Zuckerbausteine sowie ihre entsprechenden Derivate.

Weiterhin werden zu den für Formel I genannten Kohlehydraten Saccharide gerechnet, die zusätzlich einen Aminorest, einen Thiolrest oder einen aus zwei benachbarten OH-Gruppen und einem Aldehyd bzw. einem Keton gebildeten cyclischen Acetalrest enthalten.

Die Verknüpfung eines Saccharids mit dem Sauerstoffatom in 13β-Position der Verbindungen der Struktur I wie auch die Verknüpfung zwischen den Zuckerresten eines Di- oder Trisaccharids kann als α-oder β-Anomeres erfolgen. Die vorliegende Erfindung bezieht sich auf beide Bindungsarten.

Zur Bildung eines cyclischen Acetals an einem Zuckermolekül eignen sich einfache Aldehyde wie Acetaldehyd, Propionaldehyd, Butyraldehyd, Benzaldehyd oder Ketone wie Acetophenon, Cyclopentanon, Cyclohexanon, Cycloheptanon, Fluorenon, Methylethylketon, vor allem aber Aceton unter Bildung entsprechender Acetonide.

Folgende Untergruppen von Verbindungen der Formel I sind auf Grund ihrer ausgeprägten parasitiziden und insektiziden Wirkung besonders bevorzugt:

Gruppe Ia: Verbindungen der Formel I, worin R für eine Kohlenhydratgruppe $-A-(B)_m-(C)_n$ steht, wobei A einen in 1'-Stellung verknüpften Kohlenhydratrest repräsentiert, der glykosidisch an ein zweites oder drittes Kohlenhydratmolekül B und/oder C beliebiger Struktur geknüpft sein kann, wobei m und n unabhängig 0 oder 1 bedeuten; $R_1$ Wasserstoff oder eine Schutzgruppe bedeutet; und $R_2$ für Methyl, Ethyl, Isopropyl, oder sek.-Butyl steht.

Gruppe Ib: Diejenigen Verbindungen innerhalb der Untergruppe Ia, worin $R_1$ Wasserstoff bedeutet.

Gruppe Ic: Verbindungen der Formel I innerhalb der Untergruppe Ia, worin $R_1$ die Gruppe $-Si(R_5)(R_6)(R_7)$ repräsentiert, wobei $R_5$, $R_6$ und $R_7$ unabhängig voneinander für $C_1$-$C_4$-Alkyl, Benzyl oder Phenyl stehen; und $R_2$ für Methyl, Ethyl, Isopropyl oder sek.-Butyl steht.

Gruppe Id: Diejenigen Verbindungen innerhalb der Untergruppe Ic, worin $R_1$ Trimethylsilyl, tris(tert.-Butyl)-silyl, Diphenyl-tert.-butylsilyl, bis(Isopropyl)methylsilyl, Triphenylsilyl, Dimethyl-(2,3-dimethyl-2-butyl)silyl oder tert.-Butyl-dimethylsilyl bedeutet; und $R_2$ für Methyl, Ethyl, Isopropyl oder sek.-Butyl steht.

Gruppe Ie: Diejenigen Verbindungen im Umfang der Untergruppe Ia, worin $R_1$ eine Acylgruppe bedeutet.

Gruppe If: Diejenigen Verbindungen im Umfang der Untergruppe Ia, worin $R_1$ eine Acetyl oder Benzoylgruppe bedeutet.

Gruppe Ig: Verbindungen der Formel I, worin R für den Zuckerrest der Formel U

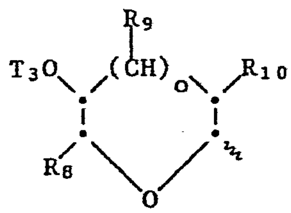 (U)

unter Einschluss seiner Stellungs-Isomeren repräsentiert, wobei o für die Zahl 0 oder 1 steht; $R_8$ Wasserstoff, Methyl oder $-CH_2-O-T_1$ bedeutet; $R_9$ und $R_{10}$ unabhängig voneinander für Wasserstoff oder $OT_2$ steht, oder an Stelle von $R_9$ und $R_{10}$ eine Doppelbindung vorliegt; $T_1$, $T_2$ und $T_3$ unabhängig voneinander Wasserstoff, Methyl, Benzyl, eine unsubstituierte oder halogensubstituierte $C_1$-$C_6$-aliphatische Acylgruppe, eine Benzoylgruppe, oder eine $C_1$-$C_6$-Alkoxycarbonylgruppe bedeuten, oder wobei $T_1$ und $T_2$ zusammen mit dem Carbonyl-Kohlenstoffatom eines aliphatischen oder aromatischen Aldehyds oder Ketons mit maximal 13 Kohlenstoffatomen ein cyclisches Acetal bilden; $R_1$ Wasserstoff oder eine Schutzgruppe bedeutet; und $R_2$ für Methyl, Ethyl, Isopropyl oder sek.-Butyl steht.

Gruppe Ih: Verbindungen der Formel I aus der Untergruppe Ig wobei $T_3$ für einen Zuckerrest der Formel U steht und $R_8$, $R_9$, $R_{10}$, $T_1$ und $T_2$ die oben angegebene Bedeutung haben.

Gruppe Ii: Verbindungen der Formel I aus der Untergruppe Ig, worin U für ein Monosaccharid steht.

Gruppe Ik: Verbindungen der Formel I aus der Untergruppe Ii worin U ein 2-Deoxy-Zuckerrest bedeutet.

Gruppe Il: Verbindungen der Formel I aus der Untergruppe Ig, worin U für ein Disaccharid steht.

Besonders bevorzugte Einzelsubstanzen der Formel I sind z.B.:

13β-O-(3,4-Di-O-acetyl-2-deoxy-L-rhamnosyl)-milbemycin D;

13β-O-(4-O-Acetyl-2,3-dideoxy-2,3-didehydro-L-rhamnosyl)-milbemycin D;

13β-O-(4-O-Acetyl-L-oleandrosyl)-milbemycin D;

13β-O-(3,4,6-Tri-O-acetyl-2-deoxy-D-galactopyranosyl)-milbemycin D;

13β-O-(3,4,6-Tri-O-acetyl-2-deoxy-D-glucopyranosyl)-milbemycin D;

13β-O-[4'-O-(L-Oleandrosyl)-L-oleandrosyl]-milbemycin D;

13β-O-[4'-O-(L-Oleandrosyl)-L-oleandrosyl]-milbemycin $A_4$;

13β-O-[4'-O-(4"-O-Acetyl-L-oleandrosyl)-L-oleandrosyl]-milbemycin $A_4$;

13β-O-[4'-O-(L-Oleandrosyl)-2,3-dideoxy-L-rhamnosyl]-milbemycin D;

13β-O-[4'-O-(3",4"-Di-O-methyl-2-deoxy-L-rhamnosyl)-2',3'-dideoxy-L-rhamnosyl]-milbemycin $A_4$;

Die vorliegende Erfindung betrifft nicht nur die Verbindungen der Formel I, sondern gleichermassen das Verfahren zu deren Herstellung. Es wurde nämlich überraschend gefunden, dass man durch Reaktion einer Verbindung der Formel Ia

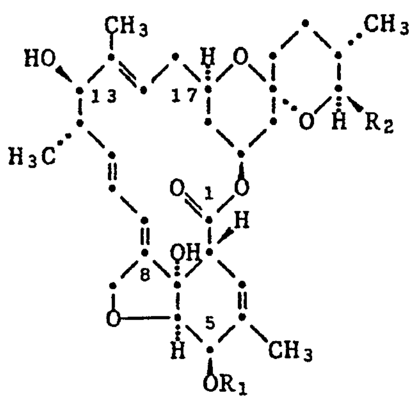

(Ia)

worin

$R_1$ eine Schutzgruppe bedeutet; und
$R_2$ für Methyl, Ethyl, Isopropyl oder sek.-Butyl steht
mit einem reaktionsfähigen Zuckerderivat zu den Verbindungen der Formel I gelangt.

Das erfindungsgemässe Verfahren bildet somit die Möglichkeit, in der 13β-Position von Milbemycin- oder 13-Deoxi-22,23-dihydro-avermectin-B1-aglykon-derivaten gezielt den unter Formel I definierten über ein Sauerstoffatom gebundenen Zuckerrest R einzuführen und damit zu hochwirksamen Parasitiziden und Insektiziden zu gelangen, die überdies für weitere Derivatisierungen eingesetzt werden können.

Die Herstellung von Verbindungen der Formel I, die an das Sauerstoffatom in der 5-Position eine Schutzgruppe tragen, stellt eine Derivatisierung der reaktionssfähigen 13β-Hydroxygruppe von 13β-Hydroxy-Milbemycin mit einem geeigneten Kohlenhydratmolekül dar und wird nach einem der in der Zuckerchemie verwendeten Verknüpfungsmethoden, z.B. nach der Koenigs-Knorr-Synthese, dem Ag-Triflat-Prozess, nach dem sogenannten Orthoester-Verfahren, der Phenylthio-Synthese oder der 2-Pyridylthio-Synthese durchgeführt.

A) Nach der Koenigs-Knorr-Synthese oder dem Silber-Triflat-Prozess lässt sich ein 13β-Hydroxy-Milbemycin der Formel ($R_1$= eine Silyl- bzw. Acylschutzgruppe) in Gegenwart eines Silbersalzes oder Quecksilber-Salzes als Kondensationsmittel mit dem einzuführenden Zuckerrest, dem Kohlehydrat A oder $A-(B)_m-(C)_n$, worin A, B, C, k und m die für Formel I gegebene Bedeutung haben und worin sämtliche OH-Gruppen geschützt sind, mit Ausnahme der durch Chlor oder Brom substituierten 1-OH-Gruppe im Temperaturbereich von -30°C bis +60°C, bevorzugt -5°C bis +30°C unter Licht-Ausschluss gewinnen. Das gewünschte Kohlehydrat kann, sofern in 13β-Position ein Rest $A-(B)_m-(C)_n$ addiert werden soll, schrittweise an ein 13β-Hydroxy-Milbemycin gebunden werden, oder es kann vorzugsweise als bereits fertig vorgebildetes Gykosid in einem Reaktionsschritt mit dem 13β-Hydroxy-Milbemycin verknüpft werden.

Als Silbersalz lässt sich frisch gefälltes $Ag_2O$ verwenden, vorzugsweise jedoch $Ag_2CO_3$ oder $CF_3$-COOAg. Besonders bevorzugt ist Silber-Trifluormethansulfonat (Ag-Triflat = $CF_3$-$SO_3Ag$), in dessen Gegenwart die Glykosidierung bereits bei Minustemperaturen schnell abläuft. Zum Aktivieren der 13β-OH-Gruppe des 13β-Hydroxy-Milbemycins und zum Neutralisieren der gegebenenfalls entstehenden $CF_3$-$SO_3H$ bzw. $CF_3$-COOH setzt man zweckmässig ein tert.-Amin (Triethylamin, Diisopropylethylamin, Diazabicycloundecan u.a.) der Reaktionslösung zu.

Sofern gewünscht wird, können die Schutzgruppen am Zuckerrest durch milde Verseifung (z.B. $NH_3/CH_3OH$) anschliessend abgespalten werden. Als Lösungsmittel kommen bei diesem Teilschritt insbesondere wasserfreie aprotische Vertreter wie Dichlormethan, Acetonitril, Benzol, Toluol, Nitromethan, Dioxan, THF, Ethylenglykoldimethylether in Frage; Diethylether ist besonders geeignet.

Das geschützte 1-Chlor- oder 1-Brom-Kohlehydrat wird in äquimolarer Menge zum 13β-Hydroxy-Milbemycin (Ia) eingesetzt, vorzugsweise jedoch in einem 1,5- bis 3-fachen Ueberschuss. Die Reaktionsdauer beträgt, um eine befriedigende Ausbeute zu erzielen, 5 bis 72 Stunden.

Anstatt des Silbersalzes lässt sich auch Hg-Cyanid oder eine Kombination von HgO mit wahlweise Hg-Chlorid oder Hg-Bromid verwenden (Helferich-Synthese).

Nach einer weiteren Variante lässt sich die Reaktivität in der 1'-Stellung des glykosidisch zu verknüpfenden Kohlehydrats, dessen weitere OH-Gruppen geschützt sein müssen, durch anfängliche Umwandlung in das 1'-Phenylthio-Derivat und anschliessende Reaktion mit DAST (= Diethylaminoschwefeltrifluorid) in absolut trockenem Dichlormethan (Molekularsieb) bei +5°C bis -30°C zum 1'-Fluorderivat erhöhen. Reaktiver als das entsprechende, bei der Koenigs-Knorr-Synthese eingesetzte 1'-Chlor- oder 1'-Brom-Derivat lässt sich das so gewonnene 1'-Fluor-Derivat des Kohlehydrat-Reaktanden mit

13β-Hydroxy-Milbemycin (Ia) in Gegenwart von $SnCl_2$ und $AgClO_4$ in einem trockenen aprotischen Lösungsmittel wie Diethylether unter Schutzgas wie Argon bei +5°C bis -30°C verknüpfen.
(J. Am. Soc. 1984, 106, 4189-4192).

B) Eine bessere Reaktion wird erzielt, wenn das in 1'-Stellung zu aktivierende, gleichermassen geschützte Kohlehydrat mit 2,2'-Dithiopyridin in trockenem Dichlormethan bei ca. 0°C und unter Argon-Atmosphäre in das 1'-S-(2-Pyridyl)-Kohlehydrat überführt wird, das mit der freien 13β-OH-Gruppe des 13β-Hydroxy-Milbemycins der Formel Ia in Gegenwart von $Pb(ClO_4)_2$ oder $AgClO_4$ als Kondensationsmittel bei -30° bis Raumtemperatur in Tetrahydrofuran (THF) als Lösungsmittel leicht unter Bildung der glykosidischen Bindung reagiert. (J. Org. Chem. 1983, 48, 3489-3493).

C) Glykosidische Verknüpfungen lassen sich auch in Gegenwart von Lewis-Säuren erzielen, wie $AlCl_3$, $AlBr_3$, $SnCl_4$, $ZnCl_2$, $BF_3$ (sowie vor allem das Etherat), wozu insbesondere acetylierte Zucker sehr geeignet sind. (Chimia 21, 1967, S. 537-538).

D) Nach der sogenannten Orthoester-Methode lassen sich glykosidische Bindungen auch durch Reaktion von 13β-Hydroxy-Milbemycin der Formel Ia mit dem zu verknüpfenden OH-geschützten Zucker in Gegenwart des Orthoesters eines niederen Alkohols erzielen, dessen eine alkoholische Komponente der Zucker-Reaktand ist.

E) Zur Herstellung von 13β-O-Glykosid-milbemycin-Derivaten, worin der Zuckerrest ein 2,3-Dideoxy-2,3-didehydro-glykopyranosid ist, kann auch das 5-O-geschützte 13β-Hydroxy-milbemycin der Formel Ia mit acetylierten Glykalen in Gegenwart von p-Toluolsulfonsäure bei Raumtemperatur umgesetzt werden (Ferrier Reaktion).

Das Verfahren zur Herstellung von 13β-O-Glykosid-Milbemycin-Derivaten der Formel, worin $R_1$, $R_2$, A, B, C, m und n die unter Formel I angegebenen Bedeutungen haben, ist gekennzeichnet im engeren Sinne durch Reaktion von 5-O-geschütztem 13β-Hydroxi-Milbemycin der Formel Ia

a) in Gegenwart eines Silbersalzes oder Quecksilber-Salzes als Kondensationsmittel mit dem einzuführenden Kohlenhydrat A oder $A-(B)_m-(C)_n$, worin A, B, C, m und n die für Formel I gegebene Bedeutung haben und worin sämtliche OH-Gruppen geschützt sind, mit Ausnahme der in 1-Stellung durch Chlor oder Brom substituierten anomeren 1-OH-Gruppe unter Licht-Ausschluss im Temperaturbereich von -30°C bis +60°C, bevorzugt -5°C bis +30°C; oder

b) in Gegenwart von $SnCl_2$ und $AgClO_4$ als Kondensationsmittel mit dem einzuführenden Kohlenhydrat A oder $A-(B)_m-(C)_n$, worin sämtliche OH-Gruppen geschützt sind, mit Ausnahme der in 1-Stellung durch Fluor substituierten anomeren 1-OH-Gruppe unter Lichtausschluss bei +5°C bis -30°C; oder

c) in Gegenwart von $Pb(ClO_4)_2$ oder $AgClO_4$ als Kondensationsmittel mit dem einzuführenden Kohlenhydrat A oder $A-(B)_m-(C)_n$, worin sämtliche OH-Gruppen geschützt sind, mit Ausnahme der in 1-Stellung durch 5-(2-Pyridyl) substituierten anomeren OH-Gruppe bei -30° bis Raumtemperatur in Tetrahydrofuran (THF) als Lösungsmittel; und sofern gewünscht, milder Verseifung der Hydroxyl-Schutzgruppen mit 25 % Ammoniak in Methanol bei 0° bis Raumtemperatur bevorzugt bei 0-5°. Nach erfolgter Glykosidierungs-Reaktion wird die Silyl-Schutzgruppe zweckmässigerweise durch Behandlung der Verbindung der Formel I mit einer verdünnten Säure wie z.B. mit 1 proz. p-Toluolsulfonsäure in Methanol mit einer wässrigen HF-Lösung in Acetonitril im Temperaturbereich -30° bis 0°C, bevorzugt bei -10° oder mit Pyridiniumfluorid in Pyridin wieder abgespalten.

Die Kohlenhydratmoleküle $A-(B)_m-(C)_n$ sind bekannt oder lassen sich analog zu den bekannten Vertretern herstellen.

Die 13β-Hydroxy-Verbindungen der Formel Ia, worin $R_1$ für eine Schutzgruppe steht, lassen sich aus Verbindungen der Formel II durch Reaktion mit Pyridiniumdichromat [PDC = $(Pyr)_2Cr_2O_7$] herstellen. Man arbeitet hierbei in Dimethylformamid (DMF) bei Temperaturen zwischen ca. -10° und +60°C. Die Verbindungen der Formel II, worin $R_1$ und $R_2$ die für die Formel I genannten Bedeutung haben sind aus der Europ. Offenlegungsschrift EP 0147 852 bekannt geworden.

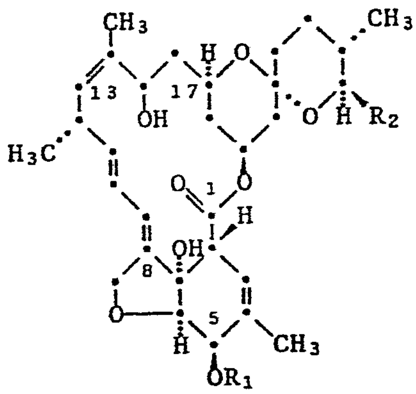

(II)

Die Verbindungen der Formel I eignen sich ausgezeichnet zur Bekämpfung von Schädlingen an Tieren und Pflanzen, darunter tierparasitären Ekto-Parasiten. Zu letzteren zählen unter der Ordnung Acarina insbesondere Schädlinge der Familien Ixodidae, Dermanyssidae, Sarcoptidae, Psoroptidae; die Ordnungen Mallophaga; Siphonaptera, Anoplura (z.B. Familie der Haemotopinidae); unter der Ordnung Diptera insbesondere Schädlinge der Familien Muscidae, Calliphoridae, Oestridae, Tabanidae, Hippoboscidae, Gastrophilidae.

Die Verbindungen I sind auch einsetzbar gegen Hygiene-Schädlinge, insbesondere der Ordnungen Diptera mit den Familien Sarcophagidae, Anophilidae, Culicidae; der Ordnung Orthoptera, der Ordnung Dictyoptera (z.B. Familie Blattidae) und der Ordnung Hymenoptera (z.B. Familie Formicidae).

Die Verbindungen I besitzen auch nachhaltige Wirksamkeit bei pflanzenparasitären Milben und Insekten. Bei Spinnmilben der Ordnung Acarina sind sie wirksam gegen Eier, Nymphen und Adulte von Tetranychidae (Tetranychus spp. und Panonychus spp.).

Hohe Aktivität besitzen sie bei den saugenden Insekten der Ordnung Homoptera, insbesondere gegen Schädlinge der Familien Aphididae, Delphacidae, Cicadellidae, Psyllidae, Loccidae, Diaspididae und Eriophydidae (z.B. die Rostmilbe auf Citrusfrüchten): Der Ordnungen Hemiptera; Heteroptera und Thysanoptera; sowie bei den pflanzenfressenden Insekten der Ordnungen Lepidoptera; Coleoptera; Diptera und Orthoptera.

Sie sind ebenfalls als Bodeninsektizid gegen Schädlinge im Erdboden geeignet.

Die Verbindungen der Formel I sind daher gegen alle Entwicklungsstadien saugender und fressender Insekten an Kulturen wie Getreide, Baumwolle, Reis, Mais, Soja, Kartoffeln, Gemüse, Früchten, Tabak, Hopfen, Citrus, Avocados und anderen wirksam.

Die Verbindungen der Formel I sind auch wirksam gegen Pflanzen-Nematoden der Arten Meloidogyne, Heterodera, Pratylenchus, Ditylenchus, Radopholus, Rizoglyphus und andere.

Besonders aber sind die Verbindungen gegen Helminthen wirksam, unter denen die endoparasitären Nematoden die Ursache schwerer Erkrankungen an Säugetieren und Geflügel sein können, z.B. an Schafen, Schweinen, Ziegen, Rindern, Pferden, Eseln, Hunden, Katzen, Meerschweinchen, Ziervögeln. Typische Nematoden dieser Indikation sind: Haemonchus, Trichostrongylus, Ostertagia, Nematodirus, Cooperia, Ascaris, Bunostonum, Oesophagostonum, Charbertia, Trichuris, Strongylus, Trichonema, Dictyocaulus, Capillaria, Heterakis, Toxocara, Ascaridia, Oxyuris, Ancylostoma, Uncinaria, Toxascaris und

Parascaris. Der besondere Vorteil der Verbindungen der Formel I ist ihre Wirksamkeit gegen solche Parasiten, die gegen Wirkstoffe auf Benzimidazol-Basis resistent sind.

Gewisse Spezies der Arten Nematodirus, Cooperia und Oesophagostomum greifen den Intestinaltrakt des Wirtstiers an, während andere der Arten Haemonchus und Ostertagia im Magen und solche der Art Dictyocaulus im Lungengewebe parasitieren. Parasiten der Familien Filariidae und Setariidae finden sich im internen Zellgewebe und den Organen, z.B. dem Herzen, den Blutgefässen, den Lymphgefässen und dem subcutanen Gewebe. Hier ist vor allem der Herzwurm des Hundes, Dirofilaria immitis, zu nennen. Die Verbindungen der Formel I sind gegen diese Parasiten hoch wirksam.

Sie sind ferner zur Bekämpfung von humanpathogenen Parasiten geeignet, unter denen als typische, im Verdauungstrakt vorkommende Vertreter solche der Arten Ancylostoma, Necator, Ascaris, Strongyloides, Trichinella, Capillaria, Trichuris und Enterobius zu nennen sind. Wirksam sind die Verbindungen vorliegender Erfindung auch gegen Parasiten der Arten Wuchereria, Brugia, Onchocerca und Loa aus der Familie der Filariidae, die im Blut, im Gewebe und verschiedenen Organen vorkommen, ferner gegen Dracunculus und Parasiten der Arten Strongyloides und Trichinella, die speziell den Gastro-Intestinalkanal infizieren.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Verbindungen der Formel I werden bei Warmblütern in Aufwandmengen von 0,01 bis 10 mg/kg Körpergewicht angewendet, über geschlossenen Kultur-Anbauflächen, in Pferchen, Stallungen oder sonstigen Räumen in Mengen von 10 g bis 1000 g pro Hektar.

Die Formulierungen, d.h. die den Wirkstoff der Formel I enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Die in der Formulierungstechnik gebräuchlichen Tenside sind u.a. in folgender Publikation beschrieben:

"Mc Cutcheon's Detergents and Emulsifiers Annual"
MC Publishing Corp., Ridgewood, New Jersey, 1982.

Die pestiziden Zubereitungen enthalten in der Regel 0,01 bis 95 %, insbesondere 0,1 bis 80 %, Wirkstoff der Formel I, 5 bis 99,99 % eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel mit 1-10'000 ppm Wirkstoffgehalt.

Ein weiterer Gegenstand vorliegender Erfindung betrifft daher Schädlingsbekämpfungsmittel, die neben üblichen Trägerstoffen und/oder Verteilungsmitteln als mindestens einen Wirkstoff eine Verbindung der Formel I enthalten.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

## Herstellungsbeispiele

### Herstellung von Ausgangs- und Zwischenprodukten

**Beispiel A1: Herstellung von 5-O-tert.Butyldimethylsilyl-13ß-hydroxy-milbemycin D und von 13ß-Hydroxy-milbemycin D (Formel Ia)**

Eine Lösung bestehend aus 286 mg (0,41 mmol) 5-O-tert.Butyldimethylsilyl-15-hydroxy-$\Delta^{13,14}$-milbemycin D und 209 mg (0,56 mmol) Pyridiniumdichromat (PDC) in 3 ml Dimethylformamid (DMF) wird 30 min bei Raumtemperatur gerührt. Anschliessend wird 1 ml Isopropanol zugegeben, 5 min weitergerührt und dann mit 50 ml Ether verdünnt. Nach weiteren 10 min wird das Gemisch durch Kieselgel filtriert und eingeengt. Bei der Chromatographie des Rohproduktes an 20 g Kieselgel (Ether/Hexan 1:2) werden 165 mg (57 %) 5-O-t-Butyldimethylsilyl-13ß-hydroxy-Milbemycin D erhalten.
$^1$H-NMR (300 MHz; $CDCl_3$; TMS):
1,59 ppm (br. s)($C_{14}CH_3$)
3,70 ppm (d; J = 10 Hz)($C_{13}H$).

105 mg (0,153 mmol) der so gewonnenen Verbindung werden mit 1 ml einer 1 %igen Lösung von p-Toluolsulfonsäure in Methanol 1 Stunde bei Raumtemperatur gerührt. Das Gemisch wird mit 20 ml Ether verdünnt, durch Kieselgel filtriert, eingeengt, und der Rückstand wird an ca. 10 g Kieselgel chromatographiert (Aceton/Dichlormethan 1:4), wobei 73 mg (83 %) 13ß-Hydroxy-Milbemycin D erhalten werden.
$^1$H-NMR (300 MHz; $CDCl_3$; TMS):
1,58 ppm (br.s)($C_{14}CH_3$)
3,71 ppm (d; J = 10 Hz)($C_{13}H$).

**Beispiel A2: Herstellung von 5-O-tert.Butyldimethylsilyl-13ß-hydroxy-milbemycin $A_4$**

Eine Lösung bestehend aus 1,06 mg (1,59 mmol) 5-O-tert.Butyldimethylsilyl-15-hydroxy-$\Delta^{13,14}$-milbemycin $A_4$ und 383 mg (1,02 mmol) Pyridiniumdichromat (PDC) in 5 ml Dimethylformamid (DMF) wird

30 min. bei Raumtemperatur gerührt. Anschliessend wird 1 ml Isopropanol zugegeben, 5 min. weitergerührt und dann mit 50 ml Ether verdünnt. Nach weiteren 10 min. wird das Gemisch durch Kieselgel filtriert und eingeengt. Nach chromatographischer Reinigung des Rohproduktes an 20 g Kieselgel (Ether/Hexan 1:2) werden 625 mg (59 %) 5-O-t-Butyldimethylsilyl-13β-hydroxy-Milbemycin A4 erhalten.

$^1$H-NMR (300 MHz; $CDCl_3$; TMS):

0,98 ppm (t; J = 7 Hz) ($\underline{CH_3}CH_2$)

1,95 ppm (br. s) ($C_{14}CH_3$)

3,69 ppm (d; J = 9 Hz) ($C_{13}H$).

Herstellung von Endprodukten der Formel I:

Beispiel H1: Herstellung von 13β-O-(3,4-Di-O-acetyl-2-deoxy-L-rhamnosyl) milbemycin D.

Zu einer Lösung von 200 mg (0.29 mmol) 5-O-tert.-Butyldimethylsilyl-13β-hydroxy-milbemycin D in 3 ml abs. THF wird unter Argon eine Lösung von 122 mg (0.38 mmol) 1-S-(2'-Pyridyl)-3,4-di-O-acetyl-2-deoxy-L-thio-rhamnose in 2 ml abs. THF und bei 0° eine Lösung von 122 mg (217 mmol) $AgClO_4$ in 1 ml abs. THF zugegeben. Das Gemisch wird 30 min. bei Raumtemperatur gerührt. Zur Aufarbeitung wird das Reaktionsgemisch bei -30° mit 2 ml Triethylamin versetzt, in Diethylether aufgenommen und filtriert. Die chromatographische Reinigung an Kieselgel (Laufmittel: Aceton/Dichlormethan 1:19) ergibt 50 mg (19 %) 5-O-tert.-Butyldimethylsilyl-13β-O-(3,4-di-O-acetyl-2-deoxy-α-L-rhamnosyl)-milbemycin D und 80 mg (31 %) 5-O-tert.-Butyl-dimethylsilyl-13β-O-(3,4-Di-O-acetyl-S-deoxy-β-L-rhamnosyl)-milbemycin D. Diese beiden Silylether werden während 4 Stunden bei -10° mit einer Lösung von 3 ml 1 % p-Toluolsulfonsäure (TsOH) in Methanol (MeOH) behandelt, wobei nach der chromatographischen Reinigung an Kieselgel (Laufmittel: Aceton/Dichlormethan 1:9) 43 mg (100 %) 13β-O-(3,4-Di-O-acetyl-2-deoxy-α-L-rhamnosyl)-milbemycin D

$^1$H-NMR (300 MHz; $CDCl_3$; TMS):

3,76 ppm (d, J = 10, 1 Hz) ($C_{13}H$)

2,01, 2,03 ppm (2s) (2 $CH_3CO_2$)
5,3-5,4 ppm (m) $C_1$'H)
und 50 mg (86 %)
13β-O-(3,4-Di-O-acetyl-2-deoxy-β-L-rhamnosyl)-milbemycin D erhalten werden.
$^1$H-NMR (300 MHz; $CDCl_3$; TMS):
3,47 ppm (d, J = 10,0 Hz) ($C_{13}$H)
2,00, 2,04 ppm (2s) (2 $CH_3CO_2$)
4,90-4,94 ppm (m) $C_1$'H).

Beispiel H2: Herstellung von 13β-O-(4-O-Acetyl-2,3-dideoxy-2,3-didehydro-L-rhamnosyl)-milbemycin D.

Zu einer Lösung von 100 mg (0.146 mmol) 5-O-tert.-Butyldimethylsilyl-13β-hydroxy-milbemycin D und 51 mg (0.238 mmol) Di-O-acetyl-L-rhamnal in 0.5 ml trockenem Dichlormethan werden bei Raumtemperatur 5 mg (0.029 mmol) p-Toluolsulfonsäure (TsOH) zugegeben. Das Gemisch wurde mit 1 ml Triethylamin versetzt, in Diethylether aufgenommen und filtriert. Die chromatographische Reinigung an Kieselgel (Laufmittel: Aceton/Dichlormethan 1:40) ergibt 79 mg (64 %) 5-O-tert.-Butyldimethylsilyl-13β-O-(4-O-acetyl-2,3-dideoxy-2,3-didehydro-L-rhamnosyl)-milbemycin D. Die Entfernung der Silylether-Schutzgruppe erfolgt durch Behandlung mit einer Lösung von 1 % p-Toluolsulfonsäure in Methanol während 3 Std. bei -10°. Nach der chromatographischen Reinigung an Kieselgel (Laufmittel: Hexan/Essigsäureethylester 2:1) werden 45 mg (66 %) 13β-O-(4-O-Acetyl-2,3-dideoxy-2,3-didehydro-L-rhamnosyl)-milbemycin D (Gemisch von α- und β-Anomer; Verhältnis ca. 3:1) erhalten.
$^1$H-NMR (300 MHz; $CDCl_3$; TMS):
2,06 und 2,07 ppm (s) ($CH_3COO$)
3,53 und 3,87 ppm (d, J = 10 Hz) ($C_{13}$H)
4,95-5,05 ppm (m) ($C_2$'H und $C_3$'H).

Beispiel H3: Herstellung von 13β-O-(4-O-Acetyl-L-oleandrosyl)-milbemycin D.

Zu einer Lösung von 330 mg (0.48 mmol) 5-O-tert.-Butyldimethylsilyl-13β-hydroxy-milbemycin D in 7 ml abs. THF wird unter Argon eine Lösung von 185 mg (0,62 mmol) 1-S-(2'-Pyridyl)-4-O-acetyl-L-thio-oleandrose in 2 ml abs. THF und bei -20° 271 mg (1.25 mmol) $AgClO_4$ in 1 ml abs. THF zugegeben. Das Gemisch wird in 15 min. auf Raumtemperatur aufgewärmt und zur Aufarbeitung mit 1 ml Triethylamin versetzt, in Diethylether gelöst und filtriert. Die chromatographische Reinigung an Kieselgel (Laufmittel: Hexan/Diethylether 1:1) und die HPLC-Trennung ebenfalls an Kieselgel (Laufmittel: Hexan/Diethylether 3:2; Druck: 30 bar) liefert 108 mg (26 %) 5-O-tert.-Butyldimethylsilyl-13β-O-(4-O-acetyl-α-L-olenadrosyl)-milbemycin D und 150 mg (36 %) 5-O-tert.-Butyldimethylsilyl-13β-O-(4-O-acetyl-β-L-oleandrosyl)-milbemycin D. Diese beiden Silylether werden während 16 Std. bei -10° mit einer Lösung von 3 ml 1 % p-Toluolsulfonsäure in Methanol behandelt, wobei nach der chromatographischen Reinigung an Kieselgel (Laufmittel: Hexan/Essigsäureethylester 5:4) 90 mg (96 %) 13β-O-(4-O-Acetyl-α-L-oleandrosyl)-milbemycin D

$^1$H-NMR (300 MHz; $CDCl_3$; TMS):
2,09 ppm (s) ($CH_3COO$)
3,34 ppm (s) ($CH_3O$)
4,47 ppm (d, J = 10 Hz) ($C_{13}H$)
4,90-4.96 ppm (m) ($C_1$'H)

und 100 mg (77 %) 13β-O-(4-O-Acetyl-β-L-oleandrosyl)-milbemycin D
$^1$H-NMR (300 MHz; $CDCl_3$; TMS)
2,08 ppm (s) ($CH_3COO$)
3,31 ppm (s) ($CH_3O$)
3,78 ppm (d, J = 10 Hz) ($C_{13}H$)
4,28-4,32 ppm (m) ($C_1$'H).

Beispiel H4: Herstellung von 13β-O-(3,4,6-Tri-O-acetyl-2-deoxy-D-galactopyranosyl)-milbemycin D.

Zu einer Lösung von 330 mg (0,48 mmol) 5-O-tert.-Butyldimethylsilyl-13β-hydroxy-milbemycin D in 6 ml abs. THF wird unter Argon zu einer Lösung von 257 mg (0,673 mmol) 1-S-(2'-Pyridyl)-3,4,6-tri-O-acetyl-D-thio-galactopyranose in 2 ml abs. THF und bei -20° 292 mg (1,34 mmol) $AgClO_4$ in 1 ml THF zugegeben. Das Gemisch wird in 15 min. auf Raumtemperatur aufgewärmt und zur Aufarbeitung mit 1 ml Triethylamin versetzt, in Diethylether gelöst und filtriert. Die chromatographische Reinigung an Kieselgel (Laufmittel: Hexan/Diethylether 8:7) liefert 140 mg (30 %) 5-O-tert.-Butyldimethylsilyl-13β-O-(3,4,6-tri-O-acetyl-2-deoxy-D-galactopyranosyl)-milbemycin D.

Die Entfernung der Silylether-Schutzgruppe erfolgt durch Behandlung mit einer Lösung von 1 % p-Toluolsulfonsäure in Methanol während 16 Std. bei -10°. Nach der chromatographischen Reinigung an Kieselgel (Laufmittel: Hexan/Essigsäureethylester 1:1) werden 95 mg (77 %) 13β-O-(3,4,6-Tri-O-acetyl-2-deoxy-D-galactopyranosyl)-milbemycin D (Gemisch von α- und β-Anomer; Verhältnis ca. 9:1) erhalten.

$^1H$-NMR (300 MHz; $CDCl_3$; TMS)
1,99. 2,02, 2,07 ppm (3s) (3 $CH_3COO$)
3,61 ppm (d, J = 10 Hz) ($C_{13}H$)
4,92-4,98 ppm (m) ($C_1$'H).

Beispiel H5: Herstellung von 13β-O-(3,4,6-Tri-O-acetyl-2-deoxy-D-glucopyranosyl)-milbemycin D.

Analog zum Beispiel H4 werden aus 330 mg (0,481 mmol) 5-O-tert.-Butyldimethylsilyl-13β-hydroxymilbemycin D durch Reaktion mit 1-S-(2'-Pyridyl)-3,4,6-tri-O-acetyl-D-thio-glucopyranose nach der Entfernung der Silyletherschutzgruppe 203 mg (50 %) der Titelverbindung (Gemisch von α- und β-Anomer; Verhältnis ca. 3:1) erhalten.

$^1H$-NMR (300 MHz, $CDCl_3$; TMS):
2,01, 2,05, 2,09 ppm (3s) (3 $CH_3COO$; α-Anomer)
2,02, 2,03, 2,07 ppm (3s) (3 $CH_3COO$; β-Anomer)
3,46 ppm (d, J = 10 Hz) ($C_{13}H$; β-Anomer)
3,60 ppm (d, J = 10 Hz) ($C_{13}H$; α-Anomer)
4,50 ppm (d, J = 7 Hz) ($C_1$'H; β-Anomer)
4,86-4,90 ppm (m) ($C_1$'H; α-Anomer).

Beispiel H6: Herstellung von 13β-O-[4'-O-(L-Oleandrosyl)-L-oleandrosyl]-milbemycin D.

Zu einer Lösung von 76 mg (0.11 mmol) 5-O-tert.-Butyldimethylsilyl-13β-hydroxy-milbemycin D und 89 mg (0.20 mmol) 1-S-(2'-Pyridyl)-4-(4'-O-Acetyl-α-L-oleandrosyl)-L-thio-oleandrose in 2 ml abs. THF wird unter Argon bei -20° eine Lösung von 130 mg (0.60 mmol) $AgClO_4$ in 1 ml abs. THF zugegeben. Das Gemisch wird 30 min. bei -20° gerührt und dann unter Zugabe von 1 ml Triethylamin in Diethylether aufgearbeitet. Die chromatographische Reinigung an Kieselgel (Laufmittel: Hexan/Diethylether 5:4) ergibt 18 mg (16 %) 5-O-tert.-Butyldimethylsilyl-13β-O-[4'-O-(4"-O-acetyl-α-L-oleandrosyl)-α-L-oleandrosyl]-milbemycin D und 15 mg (13 %) 5-O-tert.-Butyldimethylsilyl-13β-O-[4'-O-(4"-O-acetyl-α-L-oleandrosyl)-β-L-oleandrosyl]-milbemycin D sowie 40 mg (36 %) eines Gemisches dieser beiden Verbindungen. Zur Entfernung der Acetoxy- bzw. Silylether-Schutzgruppen, werden die beiden Verbindungen während 3 Tagen mit einer Lösung von 25 % Ammoniak in Methanol bei 4° und nachfolgend während 3 Std. mit 1 ml einer Lösung von 1 % p-Toluolsulfonsäure in MeOH behandelt, wobei nach der chromatographischen Reinigung an Kieselgel (Laufmittel: Dichlormethan/Aceton 5:1) 5,0 mg (33 %) 13β-O-[4'-O-(α-L-oleandrosyl)-α-L-oleandrosyl]-milbemycin D.
$^1H$-NMR (300 MHz; $CDCl_3$; TMS):
3,35, 3,41 ppm (2s) (2 $OCH_3$)
3,46 ppm (d, J = 10 Hz) ($C_{13}H$)
4,88-4,92 ppm (m) ($C_1$'H)
5,32-5,40 ppm (m) ($C_1$"H)
und 5,2 mg (41 %)

13β-O-[4'-O-(α-L-oleandrosyl)-β-L-oleandrosyl]-milbemycin D erhalten werden.

$^1$H-NMR (300 MHz; $CDCl_3$; TMS):

3,33, 3,38 ppm (2s) (2 $OCH_3$)

3,79 ppm (d, J = 10 Hz) ($C_{13}H$)

4,28-4,36 ppm (m) ($C_1$'H)

5,34-5,42 ppm (m) ($C_1$"H).

Beispiel H7: Herstellung von 13β-O-[4'-O-(α-L-Oleandrosyl)-α-L-oleandrosyl]-milbemycin $A_4$.

Analog zu Beispiel H6 wird ausgehend von 270 mg (0,40 mmol) 5-O-tert.-Butyldimethylsilyl-13β-hydroxymilbemycin $A_4$ 37 mg (11 %) der Titelverbindung erhalten.

$^1$H-NMR (300 MHz; $CDCl_3$; TMS):

3,35, 3,41 ppm (2s) (2 $OCH_3$)

3,45 ppm (d, J = 10 Hz) ($C_{13}H$)

4,86-4,92 ppm (m) ($C_1$'H)

5,32-5,40 ppm (m) ($C_1$"H).

Beispiel H8: Herstellung von 13β-O-[4'-O-(4"-O-Acetyl-α-L-oleandrosyl]-milbemycin $A_4$.

Eine Lösung von 34 mg (0,034 mmol) 5-O-tert.-Butyldimethylsilyl-13β-O-[4'-O-(4"-O-acetyl-α-L-oleandrosyl)-α-L-oleandrosyl]-milbemycin $A_4$ (Zwischenprodukt im Beispiel H7) in 2 ml abs. THF wird bei 0° mit 60 µl (0.06 mmol) einer 1M Lösung von Tetrabutylammoniumfluorid ($Bu_4NF$) versetzt und 16 Std. bei 0° gerührt. Die chromatographische Reinigung an Kieselgel (Laufmittel: Aceton/Dichlormethan 3:17) ergibt 16 mg (53 %) 13β-O-[4'-O-(4"-O-Acetyl-α-L-oleandrosyl)-α-L-oleandrosyl]-milbemycin $A_4$.

$^1$H-NMR (300 MHz; $CDCl_3$; TMS):

2,09 ppm (s) ($CH_3COO$)

3,35, 3,36 pm (2s) (2 $CH_3O$)

3,46 ppm (d, J = 10 Hz) ($C_{13}H$)

4,86-4,94 ppm (m) ($C_1$'H)

5,34-5,40 ppm (m) ($C_1$"H).

Beispiel H9: Herstellung von 13β-O-[4'-O-(3",4"-Di-O-methyl-2"-deoxy-α-L-rhamnosyl)-2',3'-dideoxy-L-rhamnosyl]-milbemycin $A_4$

Zu einer Lösung von 200 mg (0,299 mmol) 5-O-tert.-Butyldimethylsilyl-13β-hydroxy-milbemycin $A_4$ und 130 mg (0,359 mmol) 1-S-(2'-Pyridyl)-4-(3',4'-Di-O-methyl-2-deoxy-α-L-rhamnosyl)-2,3-dideoxy-L-thio-rhamnose in 5 ml abs. THF wird unter Argon bei -20° eine Lösung von 93 mg (0.43 mmol) $AgClO_4$ in 1 ml abs. THF gegeben. Das Gemisch wird 30 min. bei -20° gerührt und dann unter Zugabe von 1 ml Triethylamin in Diethylether aufgearbeitet. Die chromatographische Reinigung an Kieselgel (Laufmittel: Hexan/Diethylether 2:1) ergibt 180 mg (65 %) 5-O-tert.-Butyldimethylsilyl-13β-O-[4'-O-(3",4"-di-O-methyl-2"-deoxy-α-L-rhamnosyl)-2',3'-dideoxy-L-rhamnosyl]-milbemycin $A_4$. 68 mg (0.074 mmol) dieses Silylethers werden während 3.5 Std. bei -10° mit 1 ml einer Lösung von 1 % p-Toluolsulfonsäure in Methanol behandelt, wobei nach der chromatographischen Reinigung an Kieselgel (Laufmittel: Dichlormethan/Aceton 9:1) 57 mg (96 %) 13β-O-[4'-O-(3",4"-Di-O-methyl-2"-deoxy-α-L-rhamnosyl)-2',3'-dideoxy-L-rhamnosyl]-milbemycin $A_4$ (Gemisch von 1'-α- und 1'β-Anomer, Verhältnis ca. 2:1) anfallen.

$^1$H-NMR (300 MHz; $CDCl_3$; TMS):

3,42, 3,44, 3,54, 3,55 ppm (4s) ($OCH_3$)

3,80 ppm (d, J = 10 Hz) ($C_{13}$H)

4,28-4,36 ppm (m) ($C_1$'H; β-Anomer)

4,74-4,80 ppm (m) ($C_1$'H; α-Anomer)

5,30-5,40 ppm (m) ($C_1$"H).

Beispiel H10: Herstellung von 13β-O-[4'-O-(α-L-Oleandrosyl)-2,3-dideoxy-L-rhamnosyl]-milbemycin D.

246 mg (0.358 mmol) 5-O-tert.-Butyldimethylsilyl-13β-hydroxy-milbemycin D werden mit 167 mg (0,406 mmol) 1-S-(2'-Pyridyl)-4-(4'-O-Acetyl-α-L-oleandrosyl)-L-thio-2,3-dideoxy-rhamnose und 114 mg (0.527 mmol) $AgClO_4$ analog zum Beispiel H6 umgesetzt. Es werden 54 mg (18 %) der Titelverbindung erhalten (Gemisch von 1'α-und 1'β-Anomer; Verhältnis ca. 1:1).

$^1$H-NMR (300 MHz; $CDCl_3$; TMS):

3,38, 3,40 ppm (2s) ($OCH_3$)

3,80 ppm (d, J = 10 Hz) ($C_{13}H$)

4,28-4,36 ppm (m) ($C_1$'H; β-Anomer)

4,76-4,80 ppm (m) ($C_1$'H; α-Anomer)

5,30-5,40 ppm (m) ($C_1$"H).

Analog zu den beschriebenen Arbeitsweisen lassen sich auch die nachfolgend genannten Vertreter der Formel I herstellen.

Tabelle 1

Typische Vertreter von Verbindungen der Formel I, worin R für die Gruppe

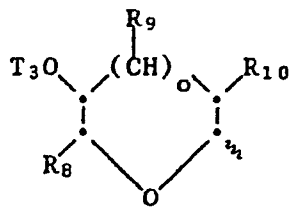

,

$R_1$ für Wasserstoff steht und $R_2$ die oben angegebene Bedeutung hat, sind: (Ac steht jeweils für Acetyl)

| Verb. Nr. | R | $R_2$ | Herst. beisp. |
|---|---|---|---|
| 1.1<br>1.2<br>1.3<br>1.4 | 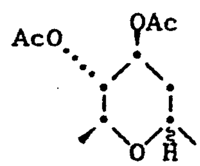 | $CH_3$<br>$C_2H_5$<br>iso-$C_3H_7$<br>sec.-$C_4H_9$ | H1 |
| 1.5<br>1.6<br>1.7<br>1.8 | 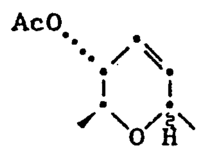 | $CH_3$<br>$C_2H_5$<br>iso-$C_3H_7$<br>sec.-$C_4H_9$ | H2 |
| 1.9<br>1.10<br>1.11<br>1.12 | 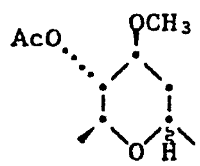 | $CH_3$<br>$C_2H_5$<br>iso-$C_3H_7$<br>sec.-$C_4H_9$ | H3 |
| 1.13<br>1.14<br>1.15<br>1.16 | 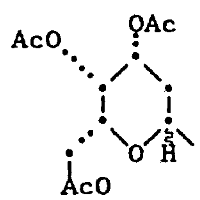 | $CH_3$<br>$C_2H_5$<br>iso-$C_3H_7$<br>sec.-$C_4H_9$ | H4 |
| 1.17<br>1.18<br>1.19<br>1.20 | 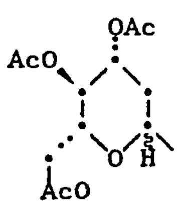 | $CH_3$<br>$C_2H_5$<br>iso-$C_3H_7$<br>sec.-$C_4H_9$ | H5 |

Tabelle 1 (Fortsetzung)

| Verb. Nr. | R | $R_2$ | Herst. beisp. |
|---|---|---|---|
| 1.21<br>1.22<br>1.23<br>1.24 | 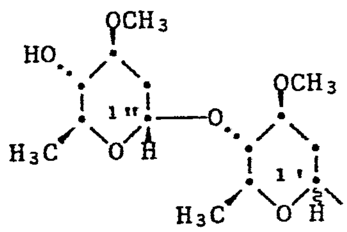 | $CH_3$<br>$C_2H_5$<br>iso-$C_3H_7$<br>sec.-$C_4H_9$ | H7<br>H6 |
| 1.25<br>1.26<br>1.27<br>1.28 | 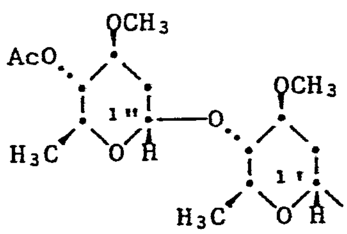 | $CH_3$<br>$C_2H_5$<br>iso-$C_3H_7$<br>sec.-$C_4H_9$ | H8 |
| 1.29<br>1.30<br>1.31<br>1.32 | 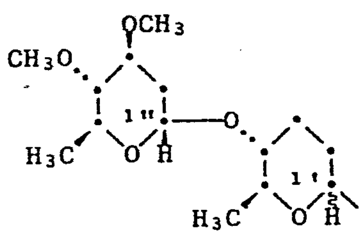 | $CH_3$<br>$C_2H_5$<br>iso-$C_3H_7$<br>sec.-$C_4H_9$ | H9 |
| 1.33<br>1.34<br>1.35<br>1.36 | 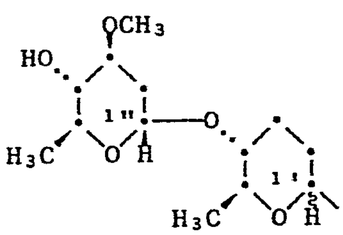 | $CH_3$<br>$C_2H_5$<br>iso-$C_3H_7$<br>sec.-$C_4H_9$ | H10 |

Formulierungsbeispiele für den Wirkstoff der Formel I

(% = Gewichtsprozent)

| Spritzpulver | a) | b) | c) |
|---|---|---|---|
| Wirkstoff aus den Tabellen | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |

| | | | |
|---|---|---|---|
| Octylphenolpolyethylenglykol-ether (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

Emulsions-Konzentrat

| | |
|---|---|
| Wirkstoff aus den Tabellen | 10 % |
| Octylphenolpolyethylenglykolether (4-5 Mol AeO) | 3 % |
| Ca-Dodecylbenzolsulfonat | 3 % |
| Ricinusölpolyglykolether (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| Stäubemittel | a) | b) |
|---|---|---|
| Wirkstoff aus den Tabellen | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

Extruder Granulat

| | |
|---|---|
| Wirkstoff aus den Tabellen | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

Tabellen bzw. Boli

| | | |
|---|---|---|
| I | Ein Wirkstoff aus den Tabellen | 33,0 % |
| | Methylcellulose | 0,80 % |
| | Kieselsäure hochdispers | 0,80 % |
| | Maisstärke | 8,40 % |

Methylcellulose in Wasser einrühren und quellen lassen; Kieselsäure in die Quellung einrühren und homogen suspendieren. Wirkstoff und Maisstärke mischen. In diese Mischung die wässrige Suspension einarbeiten und zu einem Teig kneten. Diese Masse durch ein Sieb (Maschenweite 12 M) granulieren und dann trocknen.

| | | |
|---|---|---|
| II | Milchzucker krist. | 22,50 % |
| | Maisstärke | 17,00 % |
| | mikrokrist. Cellulose | 16,50 % |
| | Magnesiumstearat | 1,00 % |

Alle 4 Hilfsstoffe gut mischen

Phasen I und II mischen und zu Tabletten oder Boli verpressen.

Falls die Verbindungen der Formel I bzw. entsprechende Mittel zur Bekämpfung von endoparasitären Nematoden, Cestoden und Trematoden bei Haus- und Nutztieren, wie Rindern, Schafen, Ziegen, Katzen und Hunden, verwendet werden, können sie den Tieren sowohl als Einzeldosis wie auch wiederholt verabreicht werden, wobei die einzelnen Gaben je nach Tierart vorzugsweise zwischen 0,1 und 10 mg pro kg Körpergewicht betragen. Durch eine protrahierte Verabreichung erzielt man in manchen Fällen eine bessere Wirkung oder man kann mit geringeren Gesamtdosen auskommen. Der Wirkstoff bzw. die ihn enthaltenden Mittel können auch dem Futter oder den Tränken zugesetzt werden. Das Fertigfutter enthält die Wirkstoffkombinationen

vorzugsweise in einer Konzentration von 0,005 bis 0,1 Gew. %. Die Mittel können in Form von Lösungen, Emulsionen, Suspensionen, Pulver, Tabletten, Bolussen oder Kapseln peroral den Tieren verabreicht werden. Soweit die physikalischen und toxikologischen Eigenschaften von Lösungen oder Emulsionen dies zulassen, können die Verbindungen der Formel I bzw. die enthaltende Mittel an Tieren auch beispielsweise subcutan injiziert, intraruminal verabreicht oder mittels der Pour-on-Methode auf den Körper der Tiere appliziert werden. Ferner ist eine Verabreichung des Wirkstoffs an die Tiere auch durch Lecksteine (Salz) oder Molasse-Blöcke möglich.

## Biologische Beispiele

### B-1. Insektizide Frassgift-Wirkung bei Spodoptera littoralis

Eingetopfte Baumwollpflanzen im 5-Blatt-Stadium werden mit einer acetonisch/wässrigen Versuchslösung besprüht, die 3, 12,5 oder 50 ppm der zu prüfenden Verbindung enthält.

Nach dem Antrocknen des Belags werden die Pflanzen mit ca. 30 Larven ($L_1$-Stadium) von Spodoptera littoralis besetzt. Pro Versuchsverbindung und pro Test-Spezies verwendet man zwei Pflanzen. Der Versuch wird bei ca. 24°C und 60 % relativer Luftfeuchtigkeit durchgeführt. Auswertungen und Zwischenauswertungen auf moribunde Tiere, Wachstum und Larven und Frassschäden erfolgen nach 24 Stunden, 48 Stunden und 72 Stunden.

Die Verbindungen der Formeln I, z.B. die Verbindungen Nr. 1.30 und 1.35, erzielten bereits bei einer Wirkstoffkonzentration von 3 ppm eine vollständige Abtötung nach 24 Stunden.

### B-2. Wirkung gegen pflanzenschädigende Akariden OP-sensible Tetranychus urticae

Die Primärblätter von Bohnenpflanzen (Phaseolus vulgaris) werden 16 Stunden vor dem Versuch mit einem durch T. urticae infestierten, aus einer Massenzucht stammenden Blattstück belegt. Die so mit allen Milben-Stadien infestierten Pflanzen werden nach Entfernung des

Blattstücks mit einer Versuchslösung bis zur Tropfnässe besprüht, die 0,8 ppm der zu prüfenden Verbindung enthält. Die Temperatur in der Gewächshauskabine beträgt ca. 25°C.

Nach sieben Tagen wird unter dem Binokular auf Prozentsatz mobiler Stadien (Adulte und Nymphen) und auf vorhandene Eier ausgewertet.

Die Verbindungen der Formel I aus den Tabellen, wie z.B. die Verbindungen Nr. 1.3 und 1.11 erzielten bereits bei einer Wirkstoffkonzentration von 0,8 ppm vollständige Abtötung.

B-3. Wirkung gegen $L_1$-Larven von Lucilia sericata

1 ml einer wässrigen Suspension der zu prüfenden Aktivsubstanz werden so mit 3 ml eines speziellen Larvenzuchtmediums bei ca. 50°C vermischt, dass ein Homogenisat von wahlweise 100 ppm oder 10 ppm Wirkstoffgehalt entsteht. In jede Reagenzglas-Probe werden ca. 30 Lucilia-Larven ($L_1$) eingesetzt. Nach 4 Tagen wird die Mortalitätsrate bestimmt. Alle Verbindungen der Formel I aus den Herstellungsbeispielen erzielten mit 100 ppm eine Wirkung von 100 %. Bei einer Verdünnung auf 10 ppm erzielten die Verbindungen Nr. 1.3, 1.7, 1.11, 1.15, 1.19, 1.23, 1.26, 1.30 und 1.35 ebenfalls noch volle Wirkung.

B-4. Akarizide Wirkung gegen Boophilus microplus (Biarra-Stamm

Auf einer PVC-Platte wird waagrecht ein Klebstreifen so befestigt, das darauf 10 mit Blut vollgesogene Zecken-Weibchen von Boophilus microplus (Biarra-Stamm) nebeneinander in einer Reihe mit dem Rücken aufgeklebt werden können. Jeder Zecke wird mit einer Injektionsnadel 1 µl einer Flüssigkeit injiziert, die eine 1:1-Mischung von Polyethylenglykol und Aceton darstellt und in der eine bestimmte Wirkstoffmenge von wahlweise 1, 0,5 oder 0,1 µl pro Zecke gelöst ist. Kontrolltiere erhalten eine wirkstofffreie Injektion. Nach der Behandlung werden die Tiere unter Normalbedingungen in einem Insektarium bei ca. 28°C und 80 % relativer Luftfeuchtigkeit gehalten, bis die Eiablage erfolgt und die Larven aus den Eiern der Kontrolltiere geschlüpft sind.

Die Aktivität einer geprüften Substanz wird mit der $IR_{90}$ bestimmt, d.h. es wird jene Wirkstoffdosis ermittelt, bei der noch nach 30 Tagen 9 von 10 Zeckenweibchen (= 90 %) Eier ablegen, die nicht schlupffähig sind.

Die Verbindungen der Formeln I aus den Tabellen, wie z.B. die Verbindungen Nr. 1.3, 1.23, 1.26, 1.30 und 1.35 erzielen eine $IR_{90}$ von 0,5 µg.

B-5. Versuch an mit Nematoden (Haemonchus concortus und Trichostrongylus colubriformis) infizierten Schafen

Der Wirkstoff wird als Suspension formuliert mit einer Magensonde oder durch Injektion in den Pansen eines Schafes gegeben, das mit Haemonchus concortus und Trychostrongylus colubriformis künstlich infiziert worden ist. Pro Dosis werden 1 bis 3 Tiere verwendet. Jedes Schaf wird nur einmal mit einer einzigen Dosis behandelt, und zwar wahlweise mit 0,5 mg oder 0,2 mg/kg Körpergewicht. Die Evaluierung erfolgt durch Vergleich der Anzahl der vor und nach Behandlung im Kot der Schafe ausgeschiedenen Wurmeier.

Gleichzeitig und gleichartig infizierte aber unbehandelte Schafe dienen als Kontrolle. Schafe, die mit einer der Verbindungen der Formeln I bei 1 mg/kg behandelt wurden, zeigten im Vergleich zu unbehandelten, aber infizierten Vergleichsgruppen keinen Nematodenbefall (= komplette Reduktion der Wurmeier im Kot). Selbst bei 0,2 mg erzielten die Verbindungen Nr. 1.22, 1.23 und 1.35 noch volle Wirkung

B-6. Kontaktwirkung auf Aphis craccivora

Erbsenkeimlinge, die mit sämtlichen Entwicklungsstadien der Laus infiziert sind, werden mit einer aus einem Emulsionskonzentrat hergestellten Wirkstofflösung besprüht, die wahlweise 50 ppm, 25 ppm oder 12,5 ppm Wirkstoff enthält. Nach 3 Tagen wird auf mehr als 80 % tote bzw. abgefallene Blattläuse ausgewertet. Nur bei dieser Wirkungshöhe wird ein Präparat als wirksam eingestuft.

Die Verbindungen der Formel I aus den Tabellen, wie z.B. die Verbindungen Nr. 1.3, 1.11 und 1.35 erzielten bei einer Konzentration von 12,5 ppm eine vollständige Abtötung (= 100 %).

B-7. Larvizidwirkung gegen Aedes aegypti

Auf die Oberfläche von 150 ml Wasser, das sich in einem Behälter befindet, wird soviel einer 0,1 %igen acetonischen Lösung des Wirkstoffes pipettiert, dass Konzentrationen von wahlweise 10 ppm, 3,3 ppm und 1,6 ppm erhalten werden. Nach Verdunsten des Acetons wird der Behälter mit ca. 30-40 3 Tage alten Aedes-Larven beschickt. Nach 1, 2 und 5 Tagen wird die Mortalität geprüft.

Die Verbindungen der Formeln I aus den Tabellen, wie z.B. die Verbindungen Nr. 1.7, 1.11, 1.23, 1.30 und 1.35 bewirkten in diesem Test bei einer Konzentration von 1,6 ppm bereits nach einem Tag eine vollständige Abtötung sämtlicher Larven.

Patentansprüche

1. Verbindungen der Formel I

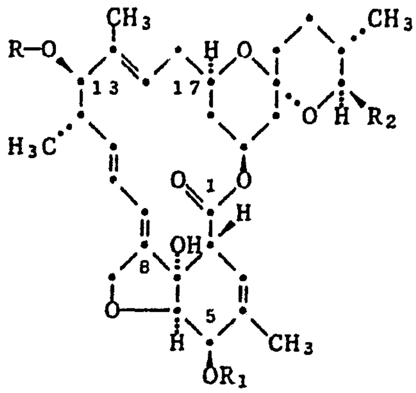

(I)

worin

$R_1$ Wasserstoff oder eine Schutzgruppe bedeutet;

$R_2$ für Methyl, Ethyl, Isopropyl oder sek.-Butyl steht; und

R für einen Zuckerrest steht.

2. Verbindungen der Formel I nach Anspruch 1, worin
$R_1$ Wasserstoff oder eine Schutzgruppe bedeutet;
$R_2$ für Methyl, Ethyl, Isopropyl oder sek.-Butyl steht; und
R für eine Kohlenhydratgruppe $-A-(B)_m-(C)_n$ steht,
wobei A einen in 1'-Stellung verknüpften Kohlenhydratrest bedeutet, der glykosidisch an ein zweites oder drittes Kohlenhydratmolekül B und/oder C beliebiger Struktur geknüpft sein kann, wobei m und n unabhängig voneinander 0 oder 1 bedeuten.

3. Verbindungen der Formel I nach Anspruch 2, worin
$R_1$ Wasserstoff oder eine Schutzgruppe bedeutet; $R_2$ für Methyl, Ethyl, Isopropyl oder sek.-Butyl steht; und
R für ein Mono-, Di- oder Trisaccharid steht, ausgewählt aus der Reihe:
Glucose, Fructose, Altrose, Mannose, Sorbose, Gulose, Idose, Allose, Galactose, Ribose, Rhamnose, Arabinose, Xylose, Lyxose, Erythrose, Threose, Thamnose, Oleandrose, Altrose, Talose, Methylglukose,

Trimethylglukose, Tetraacetylglukose 2-Deoxy-glukose, 2-Deoxy-galactose, 2-Deoxy-Rhamnose, 2-Deoxy-ribose, Lactose, Maltose, Cellobiose, Melibiose, Gentiobiose und Oleandryl-oleandrose.

4. Verbindungen der Formel I nach einem der Ansprüche 1 bis 3, worin $R_1$ Wasserstoff bedeutet, $R_2$ für Methyl Ethyl, Isopropyl oder sek.-Butyl steht und R einen Zuckerrest repräsentiert.

5. Verbindungen der Formel I nach einem der Ansprüche 1 bis 3, worin R für einen der angegebenen Zuckerreste steht; $R_1$ die Gruppe $-Si(R_5)(R_6)(R_7)$ repräsentiert, wobei $R_5$, $R_6$ und $R_7$ unabhängig voneinander für $C_1$-$C_4$-Alkyl, Benzyl oder Phenyl stehen; und $R_2$ für Methyl, Ethyl, Isopropyl oder sek.-Butyl steht.

6. Verbindungen der Formel I nach Anspruch 5, worin R für einen Zuckerrest steht; $R_1$ Trimethylsilyl, tris(tert.-Butyl)-silyl, Diphenyl-tert.-butylsilyl, bis(Isopropyl)methylsilyl, Triphenylsilyl, Dimethyl-(2,3-dimethyl-2-butyl)silyl oder tert.-Butyldimethylsilyl bedeutet; und $R_2$ für Methyl, Ethyl, Isopropyl oder sek.-Butyl steht.

7. Verbindungen der Formel I nach einem der Ansprüche 1 bis 3, worin $R_1$ für eine Acylgruppe steht; $R_2$ für Methyl, Ethyl, Isopropyl oder sek.-Butyl steht; und R einen der angegebenen Zuckerreste repräsentiert.

8. Verbindungen der Formel I nach Anspruch 7, worin $R_1$ für Acetyl oder Benzoyl steht; $R_2$ für Methyl, Ethyl, Isopropyl oder sek.-Butyl steht; und R einen der angegebenen Zuckerreste repräsentiert.

9. Verbindungen der Formel I nach Anspruch 1, worin R für den Zuckerrest der Formel U

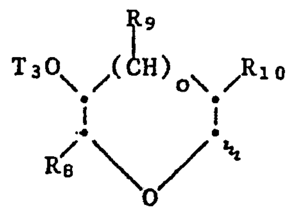

(U)

unter Einschluss seiner Stellungs-Isomeren repräsentiert, wobei o für die Zahl 0 oder 1 steht; $R_8$ Wasserstoff, Methyl oder $-CH_2-O-T_1$ bedeutet; $R_9$ und $R_{10}$ unabhängig voneinander für Wasserstoff oder $OT_2$ steht, oder an Stelle von $R_9$ und $R_{10}$ eine Doppelbindung vorliegt; $T_1$, $T_2$ und $T_3$ unabhängig voneinander Wasserstoff, Methyl, Benzyl, eine unsubstituierte oder halogensubstituierte $C_1$-$C_6$-aliphatische Acylgruppe, eine Benzoylgruppe, oder eine $C_1$-$C_6$-Alkoxycarbonylgruppe bedeuten, oder wobei $T_1$ und $T_2$ zusammen mit dem Carbonyl-Kohlenstoffatom eines aliphatischen oder aromatischen Aldehyds oder Ketons mit maximal 13 Kohlenstoffatomen ein cyclisches Acetal bilden; $R_1$ Wasserstoff oder eine Schutzgruppe bedeutet; und $R_2$ für Methyl, Ethyl, Isopropyl oder sek.-Butyl steht.

10. Verbindungen der Formel I nach Anspruch 9, worin $T_3$ für einen Zuckerrest der Formel U steht und die übrigen Substituenten wie in Anspruch 9 definiert sind.

11. Verbindungen der Formel I nach Anspruch 9, worin U für einen Monosaccharidrest steht und die übrigen Substituenten wie in Anspruch 9 definiert sind.

12. Verbindungen der Formel I nach Anspruch 9, worin U einen 2-Deoxyzuckerrest repräsentiert und die übrigen Substituenten wie in Anspruch 9 definiert sind.

13. Verbindungen der Formel I nach Anspruch 9, worin U für einen Disaccharidrest steht und die übrigen Substituenten wie in Anspruch 9 definiert sind.

14. Eine Verbindung der Formel I nach einem der Ansprüche 1 bis 3, ausgewählt aus der Reihe:

13β-O-(3,4-Di-O-acetyl-2-deoxy-L-rhamnosyl)-milbemycin D;

13β-O-(4-O-Acetyl-2,3-dideoxy-2,3-didehydro-L-rhamnosyl)-milbemycin D;

13β-O-(4-O-Acetyl-L-oleandrosyl)-milbemycin D;

13β-O-(3,4,6-Tri-O-acetyl-2-deoxy-D-galactopyranosyl)-milbemycin D;

13β-O-(3,4,6-Tri-O-acetyl-2-deoxy-D-glucopyranosyl)-milbemycin D;

13β-O-[4'-O-(L-Oleandrosyl)-L-oleandrosyl]-milbemycin D;

13β-O-[4'-O-(L-Oleandrosyl)-L-oleandrosyl]-milbemycin $A_4$;

13β-O-[4'-O-(4"-O-Acetyl-L-oleandrosyl)-L-oleandrosyl]-milbemycin $A_4$;

13β-O-[4'-O-(L-Oleandrosyl)-2,3-dideoxy-L-rhamnosyl]-milbemycin D; und

13β-O-[4'-O-(3",4"-Di-O-methyl-2-deoxy-L-rhamnosyl)-2',3'-dideoxy-L-rhamnosyl]-milbemycin $A_4$.

15. Verfahren zur Herstellung von Verbindungen der Formel I

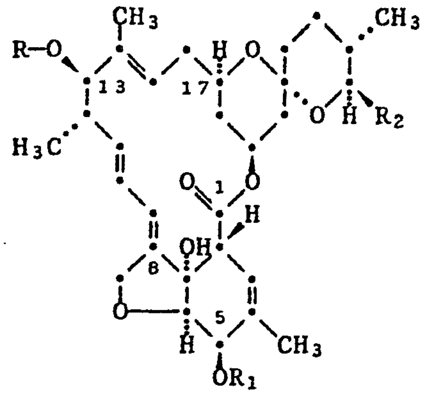

(I)

worin

$R_1$ Wasserstoff oder eine Schutzgruppe bedeutet;

$R_2$ für Methyl, Ethyl, Isopropyl oder sek.-Butyl steht; und

R für einen Zuckerrest steht, dadurch gekennzeichnet, dass man eine Verbindung der Formel Ia

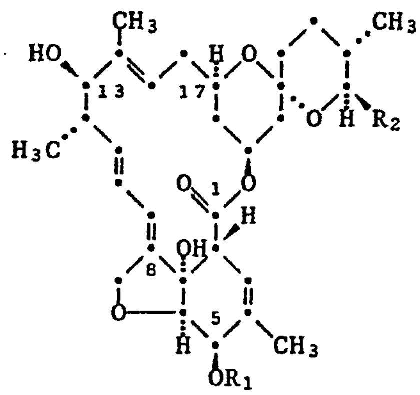

(Ia)

worin

$R_1$ eine Schutzgruppe bedeutet; und

$R_2$ für Methyl, Ethyl, Isopropyl oder sek.-Butyl steht

mit einem reaktionsfähigen Zuckerderivat umsetzt.

16. Verfahren nach Anspruch 15 zur Herstellung von Milbemycin-Derivaten der Formel I worin R und $R_1$ die in Anspruch 1 gegebene Bedeutung haben, und R die Gruppe $-A-(B)_m-(C)_n$ repräsentiert, wobei A einen in 1'-Stellung verknüpften Kohlenhydratrest bedeutet, der in 2'-Stellung eine leicht abspaltbare, über Sauerstoff gebundene

Gruppe oder eine Hydroxigruppe besitzt, und der glykosidisch an ein zweites oder drittes Kohlenhydratmolekül B und/oder C beliebiger Struktur geknüpft sein kann, wobei m und n unabhängig 0 oder 1 bedeuten, ist gekennzeichnet im engeren Sinne durch Reaktion von 13β-Hydroxi-Milbemycin der Formel I

a) in Gegenwart eines Silbersalzes oder Quecksilber-Salzes als Kondensationsmittel mit dem einzuführenden Kohlenhydrat A oder $A-(B)_m-(C)_n$, worin A, B, C, m und n die für Formel I gegebene Bedeutung haben und worin sämtliche OH-Gruppen geschützt sind, mit Ausnahme der in 1-Stellung durch Chlor oder Brom substituierten anomeren 1-OH-Gruppe unter Licht-Ausschluss im Temperaturbereich von -30°C bis +60°C, bevorzugt -5°C bis +30°C; oder

b) in Gegenwart von $Pb(ClO_4)_2$ oder $AgClO_4$ als Kondensationsmittel mit dem einzuführenden Kohlenhydrat A oder $A-(B)_m-(C)_n$, worin sämtliche OH-Gruppen geschützt sind, mit Ausnahme der in 1-Stellung durch 5-(2-Pyridyl) substituierten anomeren OH-Gruppe bei -30° bis Raumtemperatur in Tetrahydrofuran (THF) als Lösungsmittel; und sofern gewünscht, milder Verseifung der Hydroxyl-Schutzgruppen mit 25 % Ammoniak in Methanol bei 0° bis Raumtemperatur bevorzugt bei 0-5°. Nach erfolgter Glykosidierungs-Reaktion wird die Silyl-Schutzgruppe zweckmässigerweise durch Behandlung der Verbindung der Formel I mit einer verdünnten Säure wie z.B. mit 1 proz. p-Toluolsulfonsäure in Methanol mit einer wässrigen HF-Lösung in Acetonitril im Temperaturbereich -30° bis 0°C, bevorzugt bei -10° oder mit Pyridiniumfluorid in Pyridin wieder abgespalten;

und sofern gewünscht, milder Verseifung der Hydroxyl-Schutzgruppen.

17. Schädlingsbekämpfungsmittel gegen Ekto-, Endoparasiten und Insekten, dass es neben üblichen Trägerstoffen und/oder Verteilungsmitteln mindestens eine Verbindung der Formel I

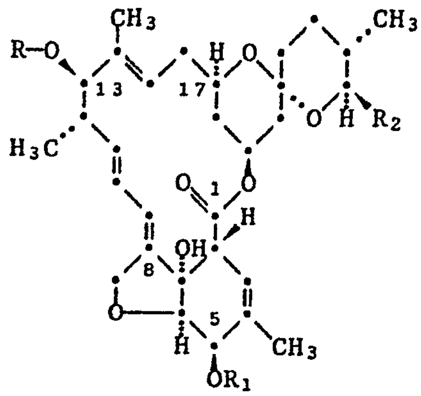

(I)

worin

$R_1$ Wasserstoff oder eine Schutzgruppe bedeutet;

$R_2$ für Methyl, Ethyl, Isopropyl oder sek.-Butyl steht; und

R für einen Zuckerrest steht, enthält.

18. Mittel gemäss Anspruch 17, dadurch gekennzeichnet, dass es als Verbindung der Formel I mindestens eine Verbindung gemäss den Ansprüchen 2 bis 11 enthält.

19. Verwendung von Verbindungen der Formel I gemäss einem der Ansprüche 1 bis 14 zur Bekämpfung von Ekto-, Endoparasiten und Insekten an Tieren und Pflanzen.

20. Verwendung gemäss Anspruch 19 zur Bekämpfung von Endoparasiten im Warmblüter.

21. Verwendung gemäss Anspruch 20, dadurch gekennzeichnet, dass es sich bei den Endoparasiten um Nematoden handelt.

22. Verfahren zur Bekämpfung von Schädlingen an Tieren und Pflanzen, dadurch gekennzeichnet, dass man eine Verbindung der Formel I gemäss einem der Ansprüche 1 bis 14 auf oder in das Tier, auf die Pflanze oder deren Lebensraum appliziert.

FO 7.5 HL/we*

Europäisches Patentamt

# EUROPÄISCHER RECHERCHENBERICHT

0235085
Nummer der Anmeldung

EP 87 81 0088

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl 4) |
|---|---|---|---|
| Y | EP-A-0 007 812 (MERCK & CO.)<br>* Seiten 53,54 * | 1,17 | C 07 D 493/22<br>A 01 N 43/90 //<br>(C 07 D 493/22<br>C 07 D 313:00<br>C 07 D 311:00<br>C 07 D 311:00<br>C 07 D 307:00 ) |
| Y,P | EP-A-0 180 539 (CIBA-GEIGY)<br>* Seiten 45-52 * | 1,17 | |
| Y,P | EP-A-0 184 173 (CIBA-GEIGY)<br>* Seiten 45-57 * | 1,17 | |
| Y,P | EP-A-0 189 159 (CIBA-GEIGY)<br>* Seiten 50-56 * | 1,17 | |
| | | | RECHERCHIERTE SACHGEBIETE (Int. Cl.4) |
| | | | C 07 D 493/00<br>A 01 N 43/00 |

Der vorliegende Recherchenbericht wurde für alle Patentansprüche erstellt.

| Recherchenort | Abschlußdatum der Recherche | Prüfer |
|---|---|---|
| DEN HAAG | 18-05-1987 | VERHULST W. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82